# EUROPÄISCHE PATENTSCHRIFT

(11) **EP 2 681 178 B1**
(45) Veröffentlichungstag und Bekanntmachung des Hinweises auf die Patenterteilung: **05.11.2014**
(21) Anmeldenummer: 12706605.8
(22) Anmeldetag: 02.03.2012
(51) Int. Cl.: C07C 7/04, C07C 7/08, C07C 7/11, C07C 11/08, C07C 11/167, C07C 9/10, B01D 3/40

(54) **VERFAHREN ZUR AUFARBEITUNG EINES BUTEN- UND/ODER BUTADIEN ENTHALTENDEN STOFFSTROMS**
PROCESS FOR WORKUP OF A STREAM COMPRISING BUTENE AND/OR BUTADIENE
PROCÉDÉ POUR TRAITER UN FLUX DE MATIÈRE CONTENANT DU BUTÈNE ET/OU DU BUTADIÈNE

(30) Priorität: 03.03.2011 EP 11156766
(43) Veröffentlichungstag der Anmeldung: 08.01.2014
(73) Patentinhaber: BASF SE, 67056 Ludwigshafen (DE)
(72) Erfinder: KOSTOVA, Albena, 68163 Mannheim (DE); BENFER, Regina, 67122 Altrip (DE); GÖTZ, Jochen, 67346 Speyer (DE); REZAI, Alireza, 68161 Mannheim (DE); MORILLO, Aristides, 67063 Ludwigshafen (DE); OLBERT, Gerhard, 69221 Dossenheim (DE); PFAB, Peter, Shaker Heights, Ohio 44122 (US); KOLIOS, Grigorios, 67435 Neustadt (DE); WEBER, Markus, 67117 Limburgerhof (DE); WECK, Alexander, 67251 Freinsheim (DE)
(86) Internationale Anmeldenummer: PCT/EP2012/053598
(87) Internationale Veröffentlichungsnummer: WO 2012/117085

(56) Entgegenhaltungen:
- EP-B1- 1 682 468
- WO-A1-2008/006879
- US-A- 3 082 271

## Beschreibung

Die Erfindung betrifft ein Verfahren zur Aufbereitung eines Buten- und/oder Butadien, Butan, Wasserstoff und/oder Stickstoff und gegebenenfalls Kohlendioxid enthaltenden Stoffstroms.

Butene und Butadien können zum Beispiel durch thermische Spaltung (steamcracking) gesättigter Kohlenwasserstoffe hergestellt werden, wobei üblicherweise von Naphta als Rohstoff ausgegangen wird. Beim Steam-Cracken von Naphta fällt ein Kohlenwasserstoff-Gemisch aus Methan, Ethan, Ethen, Acetylen, Propan, Propen, Propin, Allen, Methylallen, C₅- und höheren Kohlenwasserstoffen an.

Nachteil dieses Verfahrens zur Erzeugung von Butenen und Butadien ist, dass zwangsläufig größere Mengen an unerwünschten Koppelprodukten anfallen. Alternativ können die Butene aus Butan und das Butadien aus n-Buten durch Dehydrierung hergestellt werden.

Aus DE-A 10 2004 059 356 ist es zum Beispiel bekannt, zur Herstellung von Butadien n-Butan als Einsatzstoff einzusetzen. Zur Herstellung des Butadiens wird das n-Butan in einer Dehydrierzone durch nicht-oxidative, katalytische Dehydrierung in einen n-Butan, 1-Buten, 2-Buten, Butadien, Wasserstoff, gegebenenfalls Kohlendioxid und gegebenenfalls Wasserdampf enthaltenden Stoffstrom dehydriert. In einer zweiten Dehydrierzone werden das 1-Buten und 2-Buten weiter zu Butadien dehydriert. Der in der Dehydrierung erhaltene Stoffstrom wird anschließend komprimiert und abgekühlt, um Wasser auszukondensieren. Aus dem Reststrom, der n-Butan, Butadien, Wasserstoff, Kohlendioxid und Wasserdampf enthält, wird durch Extraktivdestillation ein im Wesentlichen Butadien enthaltender Produktstrom abgetrennt.

Ein entsprechendes Verfahren zur Herstellung von Butadien aus n-Butan ist weiterhin auch in DE-A 10 2004 061 514 beschrieben.

Nachteil der hier beschriebenen Verfahren ist, dass für die Abtrennung eines H₂-reichen Stroms in der Absorption ein anderes Lösungsmittel als in der Extraktivdestillation eingesetzt wird und somit auch eine Desorptionsstufe für das H₂-reiche Gas notwendig ist. Zudem erfolgt keine Trennung von CO₂ und H₂.

Ein weiteres Verfahren zur Aufarbeitung eines Butene enthaltenden Stoffstroms ist auch aus SU-A 1159915 bekannt. Hierbei wird der Butene enthaltende Stoffstrom zunächst einer Absorption und anschließend einer Extraktivdestillation unterzogen. Als Lösungsmittel für die Absorption und die Extraktivdestillation wird Acetonitril eingesetzt. Nachteil bei der Verwendung von Acetonitril ist, dass dieses kein Kohlendioxid löst. Die Verwendung von Acetonitril führt daher dazu, dass der Anteil an Kohlendioxid im Gas zunimmt, da das Kohlendioxid zusammen mit dem Wasserstoff in die Butandehydrierung zurückgeführt wird und nicht bei der Absorption ausgewaschen wird.

In WO-A 2006/050969 ist ein Verfahren zur Herstellung von Butadien aus n-Butan beschrieben, bei dem der Butadien enthaltende Stoffstrom aus der Dehydrierung zunächst abgekühlt wird, um Wasser auszukondensieren. In einer weiteren Kompressionsstufe und Abkühlung wird ein Kondensatstrom, der n-Butan, Butadien und Wasser enthält, erhalten. Aus dem Wasser, n-Butan und Butadien enthaltenden Stoffstrom werden n-Butan und Butadien abgetrennt und anschließend in einen im Wesentlichen aus Butadien bestehenden Produktstrom und einen n-Butan enthaltenden Rückführstrom getrennt.

Nachteil hier ist, dass die C₄-Komponenten von den Inertgasen durch eine mehrstufige Kompression und nachfolgende Kondensation abgetrennt werden. Diese Verfahrensstufe zeichnet sich durch einen hohen Energiebedarf für die Kompression auf bis ca. 30 bar aus. Die C₄-Kondensation erfolgt bei einer Temperatur von 10°C, somit wird zusätzlich eine Kälteanlage erforderlich.

Ein Verfahren zur Herstellung von 1-Buten ist zum Beispiel in EP-B 1 682 468 beschrieben. Bei dem hier beschriebenen Verfahren wird ein C₄-Strom in einem zweistufigen Verfahren durch Absorption und eine nachfolgende Desorption von Inerten abgetrennt. Nachteil des Verfahrens ist, dass das Absorptionslösungsmittel (Tetradecan) ein anderes ist als das in der Extraktivdestillation eingesetzte Lösungsmittel (NMP). Weiterhin besteht die Gefahr einer Vermischung der beiden Lösungsmittel, woraus eine reduzierte Selektivität der Absorptions- und Extraktivdestillationsstufen resultiert. Ein weiterer Nachteil ist hier, dass das Absorptionsmittel nur für C₄ selektiv ist und somit keine Abtrennung von H₂ und CO₂ erfolgt. Der Einsatz von unterschiedlichen Lösungsmitteln in dem Absorptionsschritt erfordert eine Desorption der C₄-Komponente mit Dampf bevor diese in die NMP-Extraktivdestillationskolonne weitergeleitet wird.

Aufgabe der vorliegenden Erfindung ist es, ein Verfahren zur Aufbereitung eines Buten und/oder Butadien enthaltenden Stoffstroms bereitzustellen, das sich mit weniger Aufwand und unter geringeren Kosten realisieren lässt.

Gelöst wird die Aufgabe durch ein Verfahren zur Aufbereitung eines Buten und/oder Butadien, Butan, Wasserstoff und/oder Stickstoff und gegebenenfalls Kohlendioxid enthaltenden Stoffstroms, das folgende Schritte umfasst:
(a) Absorption des Buten und/oder Butadien, Butan, Wasserstoff und/oder Stickstoff und gegebenenfalls Kohlendioxid enthaltenden Stoffstroms mit einem 80 bis 97 Gew.-% N-Methylpyrrolidon und 3 bis 20 Gew.-% Wasser enthaltenen Gemisch wobei ein N-Methylpyrrolidon, Wasser, Buten und/oder Butadien, Butan und gegebenenfalls Kohlendioxid enthaltender Stoffstrom und ein Wasserstoff und/oder Stickstoff und Butan enthaltender Stoffstrom erhalten werden,
(b) Extraktivdestillation des N-Methylpyrrolidon, Wasser, Buten und/oder Butadien, Butan und gegebenenfalls Kohlendioxid enthaltenden Stoffstroms mit einem 80 bis 97 Gew.-% N-Methylpyrrolidon und 3 bis 20 Gew.-% Wasser enthaltenden Stoffstrom, wobei der N-Methylpyrrolidon, Wasser, Buten und/oder Butadien, Butan und gegebenenfalls Kohlendioxid enthaltende Stoffstrom in einen N-Methylpyrrolidon, Wasser, Buten und/oder Butadien enthaltenden Stoffstrom sowie einen im Wesentlichen Butan und gegebenenfalls Kohlendioxid enthaltenden Stoffstrom getrennt wird.
(c) Destillation des N-Methylpyrrolidon, Wasser, Buten und/oder Butadien enthaltenden Stoffstroms in einen im Wesentlichen N-Methylpyrrolidon und Wasser enthaltenden Stoffstrom und einen Buten und/oder Butadien enthaltenden Stoffstrom.

Erfindungsgemäß werden sowohl als Lösungsmittel für die Absorption in Schritt (a) als auch als Extraktionsmittel für die Extraktion in Schritt (b) eine Mischung aus 80 bis 97 Gew.-% N-Methylpyrrolidon und 3 bis 20 Gew.-% Wasser, bevorzugt eine Mischung aus 90 bis 93 Gew.-% N-Methylpyrrolidon und 7 bis 10 Gew.-% Wasser und insbesondere eine Mischung aus 91 bis 92 Gew.-% N-Methylpyrrolidon und 8 bis 9 Gew.% Wasser, beispielsweise eine Mischung aus 91,7 Gew.-% N-Methylpyrrolidon und 8,3 Gew.-% Wasser eingesetzt.

Das erfindungsgemäße Verfahren zeichnet sich durch eine besonders effektive Abtrennung der Butene und/oder des Butadien aus. So werden Verluste durch eine unvollständige Kondensation der Butene und/oder des Butadien minimiert. Das erfindungsgemäße Verfahren verläuft bei niedrigeren Drücken als eine C₄-Kondensation und erfordert keine tiefen Temperaturen. Das Verfahren ist somit energetisch effizienter als die aus dem Stand der Technik bekannten Verfahren. Im Unterschied zu Absorptions/Desorptionsverfahren gefolgt von einer Extraktivdestillation wird bei dem erfindungsgemäßen Verfahren das gleiche Lösungsmittel eingesetzt, wobei die Zahl der notwendigen Kolonnen reduziert wird. Zudem können auch keine unterschiedlichen Lösungsmittel gemischt werden.

Die Verwendung einer Mischung von N-Methylpyrrolidon und Wasser als Lösungsmittel für die Absorption und als Extraktionsmittel in der Extraktivdestillation hat den Vorteil, dass die Siedetemperatur niedriger liegt als die Siedetemperatur bei Verwendung von reinem N-Methylpyrrolidon. Ein weiterer Vorteil ist, dass durch Zunahme des Wasseranteils in der als Lösungsmittel eingesetzten Mischung aus Wasser und N-Methylpyrrolidon die Selektivität gesteigert werden kann. Dies führt jedoch erwartungsgemäß zu einer Verringerung der Kapazität. Ein weiterer Vorteil ist die Selektivität des N-Methylpyrrolidons gegenüber Kohlendioxid. Dies ermöglicht zusätzlich zur Abtrennung der Kohlenwasserstoffe eine Abtrennung des Kohlendioxids vom Wasserstoff.

Im Rahmen der vorliegenden Erfindung wird unter Buten immer 1-Buten, 2-Buten (cis und trans) und iso-Buten verstanden. Hierbei können die verschiedenen Butene jeweils einzeln oder in beliebigen Mischungen untereinander auftreten.

Der Buten und/oder Butadien, Butan, Wasserstoff und/oder Stickstoff und gegebenenfalls Kohlendioxid enthaltende Stoffstrom kann zum Beispiel einer Butandehydrierung und/oder einer Butendehydrierung entstammen.

Ein Butenreicher Strom kann zum Beispiel als Produktstrom in einer Butandehydrierung entstehen und enthält im Allgemeinen 20 bis 70 Vol.-% Butan (n-Butan, iso-Butan), 0 bis 40 Vol.-% iso-Buten, 1 bis 15 Vol.-% 1-Buten, 1 bis 25 Vol.-% 2-Buten (cis/trans Buten), 0,1 bis 15 Vol.-% Butadien, 1 bis 70 Vol.-% Wasserdampf, 0,1 bis 40 Vol.-% Wasserstoff, 0 bis 10 Vol.-% Stickstoff, 0 bis 10 Vol.-% leichtsiedende Kohlenwasserstoffe (Ethan, Ethen, Methan, Propan, Propen) und 0 bis 5 Vol.-% Kohlenstoffoxide.

Der Butandehydrierung wird als Ausgangsstoff ein Einsatzgasstrom zugeführt, der im Allgemeinen mindestens 60 Gew.-% Butan, vorzugsweise mindestens 90 Gew.-% Butan enthält. Daneben kann er als Nebenbestandteile noch C₁-C₆-Kohlenwasserstoffe enthalten. Die Butandehydrierung kann als eine katalytische nicht-oxidative oder oxidative Dehydrierung ausgeführt werden. Das zugeführte Butan kann als n-Butan, als iso-Butan oder als eine Mischung aus n- und iso-Butan zugegeben werden. Bevorzugt wird n-Butan zugegeben.

Bei einer nicht-oxidativen katalytischen Dehydrierung wird der Butan enthaltende Einsatzgasstrom in eine Dehydrierzone eingespeist und der nicht-oxidativen katalytischen Dehydrierung unterworfen. Dabei wird n-Butan in einem Dehydrierreaktor an einem dehydrieraktiven Katalysator teilweise zu 1-Buten und 2-Buten dehydriert, wobei auch Butadien gebildet wird. Daneben fallen Wasserstoff und in geringen Mengen Methan, Ethan, Ethen, Propan und Propen an. Je nach Fahrweise der Dehydrierung können außerdem Kohlenstoffoxide (Kohlenmonoxid und Kohlendioxid), Wasser und Stickstoff im Produktgasgemisch der nicht-oxidativen katalytischen n-Butan-Dehydrierung enthalten sein. Zusätzlich liegt im Produktgasgemisch der nicht-oxidativen katalytischen Dehydrierung nicht umgesetztes Butan vor.

Die nicht-oxidative n-Butan-Dehydrierung kann grundsätzlich in allen aus dem Stand der Technik bekannten Reaktortypen und Fahrweisen durchgeführt werden. Eine vergleichsweise ausführliche Beschreibung von geeigneten Dehydrierverfahren enthält auch "Catalytica® Studies Division, Oxidative Dehydrogenation and Alternative Dehydrogenation Processes" (Study No. 412 OD, 1993, 430 Ferguson Drive, Mountain View, California, 94043-5272, USA).

Die nicht-oxidative katalytische Butan-Dehydrierung kann mit oder ohne sauerstoffhaltigem Gas als Co-Feed durchgeführt werden. Vorzugsweise wird sie als autotherme nicht-oxidative Dehydrierung unter Einspeisung von Sauerstoff als Co-Feed durchgeführt. Bei der autothermen Fahrweise wird die benötigte Wärme direkt im Reaktorsystem durch Verbrennung von Wasserstoff und/oder Kohlenwasserstoffen in Gegenwart von Sauerstoff erzeugt. Gegebenenfalls kann zusätzlich ein Wasserstoff enthaltender Co-Feed zugemischt werden. Dem Reaktionsgasgemisch der n-Butan-Dehydrierung wird in mindestens einer Reaktionszone zusätzlich Sauerstoff zugemischt und der in dem Reaktionsgasgemisch enthaltene Wasserstoff und/oder Kohlenwasserstoff zumindest teilweise verbrannt, wodurch zumindest ein Teil der benötigten Dehydrierwärme in der mindestens einen Reaktionszone direkt in dem Reaktionsgasgemisch erzeugt wird. Sauerstoff kann als Sauerstoff/Dampf-Gemisch oder als Luft-Dampf-Gemisch eingespeist werden. Durch die Verwendung von einem Sauerstoff-Dampfgemisch werden nur geringe Mengen an Inertgasen (Stickstoff) in den Gesamtprozess eingeschleust.

Im Allgemeinen wird die Menge des dem Reaktionsgasgemisch zugesetzten sauerstoffhaltigen Gases so gewählt, dass durch die Verbrennung von im Reaktionsgasgemisch vorhandenen Wasserstoff und gegebenenfalls von im Reaktionsgasgemisch vorliegenden Kohlenwasserstoffen und/oder von in Form von Koks vorliegendem Kohlenstoff die für die Dehydrierung des Butans benötigte Wärmemenge erzeugt wird. Im Allgemeinen beträgt die insgesamt zugeführte Sauerstoffmenge, bezogen auf die Gesamtmenge des Butans, 0,001 bis 0,5 mol/mol, bevorzugt 0,005 bis 0,2 mol/mol, besonders bevorzugt 0,05 bis 0,2 mol/mol.

Der zur Wärmeerzeugung verbrannte Wasserstoff ist der bei der katalytischen Butan-Dehydrierung gebildete Wasserstoff sowie gegebenenfalls dem Reaktionsgasgemisch als wasserstoffhaltiges Gas zusätzlich zugesetzter Wasserstoff. Vorzugsweise sollte soviel Wasserstoff zugegen sein, dass das Molverhältnis H₂/O₂ im Reaktionsgasgemisch unmittelbar nach der Einspeisung von Sauerstoff 1 bis 10, bevorzugt 2 bis 5 mol/mol beträgt. Dies gilt bei mehrstufigen Reaktoren für jede Zwischeneinspeisung von sauerstoffhaltigem und gegebenenfalls wasserstoffhaltigem Gas.

Die Wasserstoffverbrennung erfolgt katalytisch. Der eingesetzte Dehydrierungskatalysator katalysiert im Allgemeinen auch die Verbrennung der Kohlenwasserstoffe und von Wasserstoff mit Sauerstoff, so dass grundsätzlich kein spezieller Oxidationskatalysator erforderlich ist. Geeignete Katalysatoren sind zum Beispiel in DE-A 10 2004 061 514 beschrieben.

In einer Ausführungsform des erfindungsgemäßen Verfahrens erfolgt eine Zwischeneinspeisung von sauerstoffhaltigem Gas und von wasserstoffhaltigem Gas vor jeder Horde eines Hordenreaktors. In einer weiteren Ausführungsform des erfindungsgemäßen Verfahrens erfolgt die Einspeisung von sauerstoffhaltigem Gas und von wasserstoffhaltigem Gas vor jeder Horde außer der ersten Horde. In einer Ausführungsform ist hinter jeder Einspeisungsstelle eine Schicht aus einem speziellen Oxidationskatalysator vorhanden, gefolgt von einer Schicht aus dem Dehydrierungskatalysator. In einer weiteren Ausführungsform ist kein spezieller Oxidationskatalysator vorhanden. Die Dehydriertemperatur beträgt im allgemeinen 400 bis 1100 °C, der Druck im letzten Katalysatorbett des Hordenreaktors im Allgemeinen 0,2 bis 5 bar, bevorzugt 1 bis 3 bar. Die Belastung (GHSV) beträgt im allgemeinen 500 bis 2000 h⁻¹, bei Hochlastfahrweise auch bis zu 100 000 h⁻¹, bevorzugt 4000 bis 16 000 h⁻¹.

Die Butan-Dehydrierung wird bevorzugt in Gegenwart von Wasserdampf durchgeführt. Der zugesetzte Wasserdampf dient als Wärmeträger und unterstützt die Vergasung von organischen Ablagerungen auf den Katalysatoren, wodurch der Verkokung der Katalysatoren entgegengewirkt und die Standzeit der Katalysatoren erhöht wird. Dabei werden die organischen Ablagerungen in Kohlenmonoxid, Kohlendioxid und gegebenenfalls Wasser umgewandelt.

Ein Merkmal der nicht-oxidativen Fahrweise gegenüber einer oxidativen Fahrweise ist, dass bei der oxidativen Dehydrierung kein freier Wasserstoff in wesentlichen Mengen gebildet wird.

Ein Butadien-reicher Strom kann zum Beispiel bei einer Butendehydrierung gewonnen werden. Im Allgemeinen enthält der bei der Butendehydrierung gewonnene Butadienreiche Strom 10 bis 40 Vol.-% Butadien, 15 bis 79,9 Vol.-% n-Butan, 0 bis 10 Vol.-% 2-Buten, 0 bis 2 Vol.-% 1-Buten, 10 bis 70 Vol.-% Wasserdampf, 0 bis 10 Vol.-% leichtsiedende Kohlenwasserstoffe (Methan, Ethan, Ethen, Propan und Propen), 0,1 bis 15 Vol.-% Wasserstoff, 0 bis 10 Vol.-% Stickstoff, 0 bis 15 Vol.-% Kohlendioxid und 0 bis 7 Vol.-% Oxigenate. Oxigenate können beispielsweise Furan, Essigsäure, Maleinsäureanhydrid, Maleinsäure, Propionsäure, Acetaldehyd, Acrolein, Formaldehyd, Ameisensäure und Butyraldehyd sein. Daneben können in Spuren Acetylen, Propin und 1,2-Butadien enthalten sein.

Die Butendehydrierung kann als Einzelverfahren oder in Kombination mit einer Butandehydrierung betrieben werden. Die Butendehydrierung kann nicht-oxidativ oder oxidativ (mit einem O₂-reichem Gas als Oxidationsmittel) betrieben werden.

Bevorzugt ist die oxidative Butendehydrierung. Die oxidative Dehydrierung kann grundsätzlich in allen aus dem Stand der Technik bekannten Reaktortypen und Fahrweisen durchgeführt werden, beispielsweise im Wirbelbett, im Hordenofen, im Festbettrohr- oder Rohrbündelreaktor oder im Plattenwärmetauscherreaktor. Zur Durchführung der oxidativen Dehydrierung wird ein Gasgemisch benötigt, das ein molares Sauerstoff : n-Butene-Verhältnis von mindestens 0,5 aufweist. Bevorzugt wird bei einem Sauerstoff : n-Butene-Verhältnis von 0,55 bis 50 gearbeitet. Zur Einstellung dieses Wertes wird in der Regel das aus der nicht-oxidativen katalytischen Dehydrierung stammende Produktgasgemisch direkt oder nach einer Aufarbeitung, bei der Butene aufkonzentriert und Wasserstoff abgetrennt werden, mit reinem Sauerstoff oder einem Sauerstoffhaltigen Gas vermischt. In einer Ausführungsform enthält das Sauerstoff-haltige Gas wie im Falle der ersten (autothermen) Dehydrierstufe überwiegend Sauerstoff, mindestens 75 Vol.-%, bevorzugt mindestens 90 Vol.-%, um den Inertgasanteil in dem Produktgasstrom der Oxidehydrierung zu minimieren. In diesem Fall ist technisch reiner Sauerstoff bevorzugt. Das erhaltene Sauerstoff-haltige Gasgemisch wird dann der Oxidehydrierung zugeführt. Alternativ und bevorzugt gegenüber einem Sauerstoffhaltigen Gas, das mindestens 75 Vol.-% Sauerstoff enthält, ist es, Luft oder Magerluft mit einem Anteil von weniger als 23 Vol.-% als Sauerstoff-haltiges Gas einzusetzen. Die Oxidehydrierung wird im Allgemeinen bei einer Temperatur von 220 bis 490 °C und bevorzugt von 250 bis 450 °C durchgeführt. Man wählt einen Reaktoreingangsdruck, der ausreicht, die in der Anlage und der nachfolgenden Aufarbeitung vorhandenen Strömungswiderstände zu überwinden. Dieser Reaktoreingangsdruck liegt in der Regel bei 0,005 bis 1 MPa Überdruck, bevorzugt 0,01 bis 0,5 MPa Überdruck. Naturgemäß fällt der im Eingangsbereich des Reaktors angewendete Gasdruck weitgehend über die gesamte Schüttung aus Katalysator ab.

Der die oxidative Dehydrierung verlassende Produktgasstrom enthält neben Butadien und nicht umgesetztem n-Butan noch Wasserstoff, Kohlendioxid und Wasserdampf. Als Nebenbestandteile kann er noch Sauerstoff, Stickstoff, Methan, Ethan, Ethen, Propan und Propen sowie sauerstoffhaltige Kohlenwasserstoffe, sogenannte Oxigenate, enthalten. Im Allgemeinen enthält er praktisch kein 1-Buten mehr und nur noch geringe Anteile an 2-Buten.

In einer bevorzugten Ausführungsform wird nach der Dehydrierung aus dem Produktstrom Wasser abgetrennt. Die Abtrennung des Wassers erfolgt dabei vorzugsweise in einem Quench.

Der Butadien und/oder Buten, Butan, Wasserstoff und/oder Stickstoff und gegebenenfalls Kohlendioxid enthaltende Stoffstrom, der aus einer Oxidehydrierung oder nicht-oxidativen Dehydrierung nach teilweiser Kondensation von Wasserdampf entstammen kann, wird als Ausgangsstrom dem Schritt (a) der Aufbereitung zugeführt.

Die Absorption in Schritt (a) kann in jeder beliebigen, dem Fachmann bekannten geeigneten Absorptionskolonne durchgeführt werden. Bevorzugt wird die Absorption im Gegenstrom durchgeführt. Hierzu wird der Absorptionskolonne im unteren Bereich der Buten und/oder Butadien, Butan, Wasserstoff und/oder Stickstoff und gegebenenfalls Kohlendioxid enthaltende Stoffstrom zugeführt. Im oberen Bereich der Absorptionskolonne wird der N-Methylpyrrolidon und Wasser enthaltende Stoffstrom aufgegeben.

Am Kopf der Absorptionskolonne wird ein wasserstoffreicher und/oder stickstoffreicher Stoffstrom entnommen, der gegebenenfalls noch Reste an C₄-Kohlenwasserstoffen und gegebenenfalls Kohlenstoffoxigenate enthält. Weiterhin kann dieser Strom Inerte (beispielsweise Stickstoff) und Leichtsieder (Ethan, Ethen, Propan, Propen, Methan) enthalten. Der N-Methylpyrrolidon und Wasser enthaltende Stoffstrom kühlt den zugeführten Buten und/oder Butadien, Butan, Wasserstoff und/oder Stickstoff und gegebenenfalls Kohlendioxid enthaltenden Stoffstrom ab und absorbiert gleichzeitig bevorzugt die C₄-Komponenten und teilweise CO₂. Gegebenenfalls werden auch kleine Mengen an H₂, Inerten (N₂) und Leichtsiedern absorbiert. Dieser Strom wird am Sumpf der Absorptionskolonne abgezogen.

Die Absorption in Schritt (a) wird im Allgemeinen bei einer Sumpftemperatur im Bereich von 30 bis 160 °C, einer Kopftemperatur im Bereich von 5 bis 60°C und bei einem Druck im Bereich von 2 bis 20 bar durchgeführt. Bevorzugt wird die Absorption bei einer Sumpftemperatur im Bereich von 30 bis 100°C, bei einer Kopftemperatur im Bereich von 25 bis 50 °C und einem Druck im Bereich von 8 bis 15 bar durchgeführt.

Die Absorptionskolonne ist vorzugsweise eine Füllkörperkolonne oder eine Packungskolonne. Es ist aber auch jede beliebige andere Kolonne, zum Beispiel eine Bodenkolonne denkbar. Eine für die Absorption geeignete Kolonne weist vorzugsweise 2 bis 40 theoretische Trennstufen, bevorzugt 5 bis 25 theoretische Trennstufen auf.

Die Temperatur des N-Methylpyrrolidon und Wasser enthaltenden Stoffstroms, der der Absorptionskolonne zugeführt wird, liegt vorzugsweise bei 10 bis 70°C, bevorzugt bei 20 bis 40°C. Die Temperatur des Buten und/oder Butadien, Butan, Wasserstoff und/oder Stickstoff und gegebenenfalls Kohlendioxid enthaltenden Stoffstroms liegt vorzugsweise im Bereich zwischen 0 und 400 °C, insbesondere im Bereich zwischen 40 und 200 °C.

Das Verhältnis von eingesetztem N-Methylpyrrolidon zu Buten und/oder Butadien, Butan, Wasserstoff und/oder Stickstoff und gegebenenfalls Kohlendioxid enthaltendem Stoffstrom liegt vorzugsweise im Bereich von 2 bis 30, mehr bevorzugt im Bereich von 4 bis 30 und insbesondere im Bereich von 4 bis 15 jeweils bezogen auf die Massen der eingesetzten Stoffströme.

Der bei der Absorption anfallende N-Methylpyrrolidon, Wasser, Buten und/oder Butadien, Butan und Kohlendioxid enthaltende Stoffstrom enthält im Allgemeinen 20 bis 90 mol-% N-Methylpyrrolidon, 0 bis 50 mol-% Wasser, 0 bis 20 mol-% Butadien, 0 bis 20 mol-% 1-Buten, 0 bis 20 mol-% 2-Buten, 0 bis 50 mol-% Butan und 0 bis 20 mol-% Kohlendioxid.

Der bei der Absorption erhaltene N-Methylpyrrolidon, Wasser, Buten und/oder Butadien, Butan und Kohlendioxid enthaltende Stoffstrom wird dann in Schritt (b) einer Extraktivdestillation zugeführt.

Die Extraktivdestillation kann beispielsweise wie in Erdöl und Kohle - Erdgas - Petrochemie Band 34 (8), Seiten 343 bis 346 oder Ullmanns Enzyklopädie der technischen Chemie, Band 9, 4. Auflage 1975, Seiten 1 bis 18 beschrieben, durchgeführt werden.

Die Extraktivdestillation wird vorzugsweise bei einer Sumpftemperatur im Bereich von 100 bis 250°C, insbesondere bei einer Temperatur im Bereich von 110 bis 210 °C, einer Kopftemperatur im Bereich von 10 bis 100°C, insbesondere im Bereich von 20 bis 70°C und einem Druck im Bereich von 1 bis 15 bar, insbesondere im Bereich von 3 bis 8 bar betrieben. Die Extraktivdestillationskolonne weist vorzugsweise 5 bis 70 theoretische Trennstufen auf.

In der Extraktivdestillation wird der Buten und/oder Butadien, Butan, N-Methylpyrrolidon, Wasser und Kohlendioxid enthaltende Stoffstrom mit einem N-Methylpyrrolidon und Wasser enthaltenden Stoffstrom in einer Extraktivdestilllationszone in Kontakt gebracht. Die Extraktivdestillationszone ist im Allgemeinen in Form einer Kolonne ausgeführt, die Böden, Füllkörper oder Packungen als Einbauten enthält. Die Extraktivdestillationszone weist im Allgemeinen 10 bis 70 theoretische Böden auf, damit eine hinreichend gute Trennwirkung erzielt wird. Vorzugsweise weist die Extraktionskolonne im Kolonnenkopf eine Rückwaschzone auf. Diese Rückwaschzone dient zur Rückgewinnung des in der Gasphase enthaltenen N-Methylpyrrolidons durch flüssigen Kohlenwasserstoffrücklauf, wozu die Kopffraktion zuvor kondensiert wird. Typische Temperaturen am Kopf der Kolonne liegen zwischen 30 und 60 °C.

Der Kopfproduktstrom der Extraktivdestillationskolonne enthält Butan und Kohlenstoffdioxid und wird gasförmig abgezogen. Neben Butan und Kohlenstoffdioxid können weiterhin Butene, Wasserstoff und/oder Stickstoff und andere Leichtsieder im Kopfproduktstrom enthalten sein. In einer bevorzugten Ausführungsform wird der Kopfproduktstrom kondensiert, um CO₂ sowie gegebenenfalls enthaltenen Wasserstoff und/oder Stickstoff und Leichtsieder von Butan abzutrennen. Der flüssige Butanstrom kann zum Beispiel beim Betrieb mit einer Dehydrierung in die Dehydrierzone zurückgeführt werden.

Am Sumpf der Extraktivdestillationskolonne wird ein N-Methylpyrrolidon, Wasser, Buten und/oder Butadien und Butan enthaltender Stoffstrom gewonnen. Der N-Methylpyrrolidon, Wasser, Butadien und/oder Buten und gegebenenfalls Butan enthaltende Strom, der am Sumpf der Extraktivdestillationskolonne gewonnen wird, wird einer Destillationskolonne (c) zugeführt, in der über Kopf Butadien und/oder Buten und gegebenenfalls Butan gewonnen werden. Am Sumpf der Destillationskolonne fällt ein N-Methylpyrrolidon und Wasser enthaltender Stoffstrom an, wobei die Zusammensetzung des N-Methylpyrrolidon und Wasser enthaltenden Stoffstroms der Zusammensetzung entspricht, wie sie der Absorption und der Extraktion zugegeben wird. Der N-Methylpyrrolidon und Wasser enthaltende Stoffstrom wird aufgeteilt und zurück in die Absorption und die Extraktivdestillation geleitet. Das Verhältnis des Gemischs aus Wasser und N-Methylpyrrolidon, das der Absorption zugeführt wird zu dem Gemisch aus Wasser und N-Methylpyrrolidon, das der Extraktivdestillation zugeführt wird, liegt vorzugsweise im Bereich von 0,2 bis 20, insbesondere im Bereich von 0,3 bis 15.

Die Destillation (c) wird vorzugsweise bei einer Sumpftemperatur im Bereich von 100 bis 300 °C, insbesondere im Bereich von 150 bis 200°C und einer Kopftemperatur im Bereich von 0 bis 70°C, insbesondere im Bereich von 10 bis 50°C durchgeführt. Der Druck in der Destillationskolonne 19 liegt dabei vorzugsweise im Bereich von 1 bis 10 bara. Die Destillationskolonne 19 weist vorzugsweise 2 bis 30, insbesondere 5 bis 20 theoretische Trennstufen auf.

Alternativ zu der Entnahme des Buten und/oder Butadien und gegebenenfalls Butan enthaltenen Stoffstroms aus der Destillationsstufe über Kopf ist es alternativ auch möglich, einen Butadienreichen Strom als Seitenabzug zu entnehmen. In diesem Fall ist es möglich, den Kopfstrom an Butadien abzureichern.

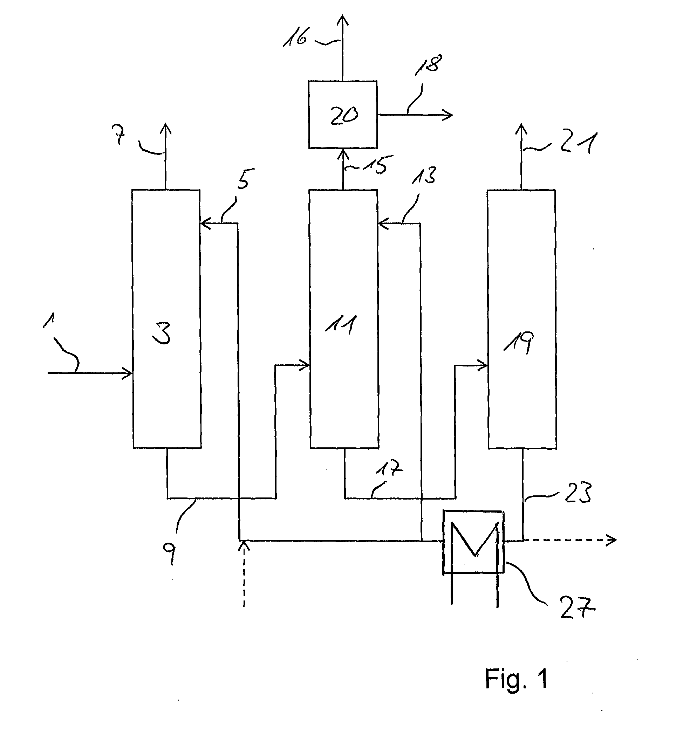

Ein Ausführungsbeispiel der Erfindung ist in der Figur dargestellt und wird in der nachfolgenden Beschreibung näher erläutert.

Ein Buten und/oder Butadien, Butan, Wasserstoff und/oder Stickstoff und gegebenenfalls Kohlendioxid enthaltender Stoffstrom 1 wird einer Absorptionskolonne 3 zugegeben. Die Zugabe erfolgt dabei im unteren Bereich der Absorptionskolonne 3. Über einen oberen Zulauf 5 wird ein N-Methylpyrrolidon und Wasser enthaltender Stoffstrom in die Absoptionskolonne 3 zugegeben, so dass der N-Methylpyrrolidon und Wasser enthaltende Stoffstrom und der Buten und/oder Butadien, Butan, Wasserstoff und/oder Stickstoff und gegebenenfalls Kohlendioxid enthaltende Stoffstrom in der Absorptionskolonne im Gegenstrom geführt werden.

Die Absorptionskolonne 3 wird bei einer Sumpftemperatur im Bereich zwischen 30 und 160 °C, bevorzugt im Bereich zwischen 30 und 100 °C, einer Kopftemperatur im Bereich von 5 bis 60°C, bevorzugt im Bereich von 25 bis 50 °C und bei einem Druck im Bereich von 2 bis 20 bar, bevorzugt im Bereich von 8 bis 15 bar betrieben.

Am Kopf der Absorptionskolonne 3 wird ein Wasserstoff und/oder Stickstoff, Reste an C₄-Kohlenwasserstoffen und Leichtsieder enthaltender Kopfstrom 7 entnommen. Ein Teil des Kopfstroms 7 wird in einer Ausführungsform in eine Butandehydrierung, aus der der zugeführte Buten und/oder Butadien, Butan, Wasserstoff und/oder Stickstoff und gegebenenfalls Kohlendioxid enthaltenen Stoffstrom stammen kann, zurückgeführt.

Am Sumpf der Absorptionskolonne 3 wird ein Buten und/oder Butadien, Butan, N-Methylpyrrolidon, Wasser und gegebenenfalls Kohlendioxid enthaltender Sumpfstrom 9 abgezogen.

Der Sumpfstrom 9 wird einer Extraktivdestillationskolonne 11 im unteren Bereich zugegeben. Im oberen Bereich der Extraktivdestillationskolonne 11 wird ein N-Methylpyrrolidon und Wasser enthaltender Stoffstrom 13 eingeleitet, der die gleiche Zusammensetzung aufweist wie der der Absorption zugeführte N-Methylpyrrolidon und Wasser enthaltende Stoffstrom. Der N-Methylpyrrolidon und Wasser enthaltende Stoffstrom 13 und der Buten und/oder Butadien, Butan, N-Methylpyrrolidon, Wasser und gegebenenfalls Kohlendioxid enthaltende Stoffstrom 9 werden ebenfalls im Gegenstrom geführt.

Aus der Extraktivdestillationskolonne 11 wird über Kopf ein Butan und Kohlendioxid enthaltender Stoffstrom 15 abgezogen. Der Stoffstrom 15 wird in einem Kondensator 20 kondensiert. Dabei entsteht ein flüssiger butanreicher Strom 16 und ein dampfförmiger CO₂-reicher Strom 18. Der butanreiche Strom 16 kann ebenfalls in die Butandehydrierung zurückgeführt werden.

Am Sumpf der Extraktivdestillationskolonne 11 wird ein N-Methylpyrrolidon, Wasser, Buten und/oder Butadien enthaltender Sumpfstrom 17 entnommen.

Der Sumpfstrom 17 wird einer Destillationskolonne 19 zugeführt, in der dieser in einen Buten und/oder Butadien enthaltenden Stoffstrom 21 der am Kopf der Destillationskolonne 19 entnommen wird, und einen N-Methylpyrrolidon und Wasser enthaltenden Stoffstrom 23, der am Sumpf der Destillationskolonne 19 entnommen wird, getrennt.

Erfindungsgemäß wird für die Absorption und für die Extraktivdestillation als Lösungsmittel ein 80 bis 97 Gew.-% N-Methylpyrrolidon und 3 bis 20 Gew.-% Wasser enthaltender Stoffstrom eingesetzt.

Der der Destillationskolonne 19 entnommene Sumpfstrom 23, der das N-Methylpyrrolidon und das Wasser enthält, wird in einem Wärmetauscher 27 abgekühlt, aufgetrennt und in die Absorptionskolonne 3 bzw. die Extraktivdestillationskolonne 11 zurückgeführt.

Die Extraktivdestillation wird vorzugsweise bei einer Sumpftemperatur im Bereich von 90 bis 250°C, insbesondere bei einer Temperatur im Bereich von 90 bis 210°C, einer Kopftemperatur im Bereich von 10 bis 100°C, insbesondere im Bereich von 20 bis 70°C und einem Druck im Bereich von 1 bis 15 bar, insbesondere im Bereich von 3 bis 8 bar betrieben. Die Extraktivdestillationskolonne weist vorzugsweise 5 bis 70 theoretische Trennstufen auf.

Die Destillation in der Destillationskolonne 19 wird vorzugsweise bei einer Sumpftemperatur im Bereich von 100 bis 300°C, insbesondere im Bereich von 150 bis 200°C und einer Kopftemperatur im Bereich von 0 bis 70°C, insbesondere im Bereich von 10 bis 50°C durchgeführt. Der Druck in der Destillationskolonne 19 liegt dabei vorzugsweise im Bereich von 1 bis 10 bara. Die Destillationskolonne 19 weist vorzugsweise 2 bis 30, insbesondere 5 bis 20 theoretische Trennstufen auf.

### Beispiele

### Beispiel 1

Einer Absorptionskolonne 3 wird im unteren Bereich ein Wasserstoff, n-Butan, Butene und Butadien enthaltender Stoffstrom 1 zugegeben. Im oberen Bereich der Absorptionskolonne wird als Lösungsmittel N-Methylpyrrolidon mit 8,3 Gew.-% Wasser aufgegeben. Am Kopf der Kolonne wird ein Wasserstoff und Butan enthaltender Kopfstrom 7 entnommen. Am Sumpf fällt ein N-Methylpyrrolidon, Wasser, Butane, Butene, Butadien und Kohlenstoffdioxid enthaltender Sumpfstrom 9 an. Als Absorptionskolonne wird eine Glockenbodenkolonne mit 70 Glockenböden mit einem Durchmesser von 50 mm eingesetzt. Der Kohlenwasserstoffe-Feedstrom wird oberhalb dem zehnten Glockenboden und das Lösungsmittel mit einem Massenstrom von 16 kg/h oberhalb dem 60sten Glockenboden zugegeben. Die Eingangstemperatur des Lösungsmittels beträgt 35°C. Die Absorption wird bei einer Kopftemperatur von 33°C, einer Sumpftemperatur von 51°C und einem Druck von 10 bara durchgeführt. Das Massenverhältnis von Lösungsmittel zu zugegebenem Wasserstoff, n-Butan, Butene und Butadien enthaltendem Stoffstrom 1 liegt bei 9,9.

Der der Absorptionskolonne entnommene Sumpfstrom 9 wird einer Extraktivdestillationskolonne 11 als Zulauf im unteren Bereich zugegeben. Im oberen Bereich wird der Extraktivdestillationskolonne ein N-Methylpyrrolidon und 8,3 Gew.-% Wasser enthaltendes Lösungsmittel zugegeben. Am Kopf der Extraktivdestillationskolonne fällt ein Kopfproduktstrom 15 an, der Butan und Kohlendioxid sowie in Spuren andere Leichtsieder enthält. Am Sumpf der Extraktivdestillationskolonne wird ein N-Methylpyrrolidon, Wasser, Butane, Butene und Butadien enthaltender Sumpfstrom 17 entnommen. Als Extraktivdestillationskolonne wird eine Packungskolonne mit 4,5 m Packung A3-1000 der Firma Montz unterteilt in 8 Packungsabschnitte eingesetzt. Oberhalb der Packung befinden sich 14 Glockenböden. Der der Absorptionskolonne entnommene Sumpfstrom 9 wird oberhalb des zweiten Packungsabschnitts zugegeben. 20 kg/h des N-Methylpyrrolidon und 8,3 Gew.-% Wasser enthaltenden Lösungsmittels werden oberhalb des achten Packungsabschnitts und unterhalb der Glockenböden zugegeben. Die Eingangstemperatur des Stroms 13 beträgt 35°C. Die Extraktivdestillationskolonne wird mit einer Sumpftemperatur von 139°C, einer Kopftemperatur von 46°C und einem Druck von 5 bara betrieben. Das Massenverhältnis von der Absorptionskolonne zugegebenem Lösungsmittel 5 zu dem der Extraktivdestillationskolonne zugegebenen Lösungsmittel 13 liegt bei 0,8.

Der Sumpfstrom aus der Extraktivdestillationskolonne wird als Zulauf einer Destillationskolonne 19 zugegeben. In der Destillationskolonne wird ein n-Butan, Butene und Butadien enthaltender Kopfstrom 21 und ein N-Methylpyrrolidon und Wasser enthaltender Sumpfstrom 23 gewonnen. Als Destillationskolonne wird eine Glockenbodenkolonne mit 48 Glockenböden eingesetzt. Oberhalb der letzten Glockenböden wird der Kolonnenkondensator installiert. Der der Extraktivdestillationskolonne entnommene Sumpfstrom 17 wird oberhalb des zweiunddreißigsten Glockenbodens zugegeben. Der Strom 21 wird als gasförmiges olefinreiches Kopfprodukt entnommen. Der Sumpfstrom 23 wird in dem Wärmetauscher 27 bis auf eine Temperatur von 35°C gekühlt und in die Kolonnen 3 und 11 zurückgeführt (Ströme 5 und 13). Die Destillationskolonne 19 wird mit einer Sumpftemperatur von 163°C, einer Temperatur unter dem Kondensator von 31°C und einem Druck von 3 bara betrieben.

Der Kopfproduktstrom 15 der Extraktivdestillationskolonne wird einem Kondensator 20 zugeführt und im Kondensator werden ein im Wesentlichen Kohlendioxid, Wasserstoff und n-Butan enthaltender Strom 18 sowie ein im wesentlichen n-Butan enthaltender Strom 16 entnommen. Der im Wesentlichen n-Butan enthaltende Strom wird bevorzugt in eine Dehydrierung zur Herstellung von Butenen und Butadien zurückgeführt.

Die Zusammensetzung und die jeweils zugeführten beziehungsweise erhaltenen Mengen der einzelnen Stoffströme sind in Tabelle 1 aufgeführt.

**Tabelle 1: Zusammensetzung der Stoffströme einer Aufarbeitung eines Buten enthaltenden Stoffstroms aus einer n-Butandehydrierung in mol-%**

| **Stoffstrom** | **1** | **7** | **15** | **21** | **16** | **18** |
|---|---|---|---|---|---|---|
| Wasserstoff | 32,63 | 88,43 | 0,89 | 0,00 | 0,00 | 12,66 |
| Kohlenstoffmonoxid | 0,20 | 0,61 | 0,00 | 0,00 | 0,00 | 0,00 |
| Ethan | 0,48 | 0,95 | 0,31 | 0,00 | 0,25 | 1,34 |
| Ethen | 0,03 | 0,04 | 0,03 | 0,00 | 0,01 | 0,13 |
| Propan | 0,94 | 1,06 | 1,62 | 0,00 | 1,69 | 2,14 |
| Propen | 0,36 | 0,06 | 0,97 | 0,00 | 0,94 | 1,47 |
| Kohlenstoffdioxid | 3,50 | 3,86 | 5,71 | 0,00 | 1,74 | 43,17 |
| Methan | 1,55 | 3,90 | 0,24 | 0,00 | 0,02 | 3,00 |
| i-Butan | 0,13 | 0,03 | 0,32 | 0,00 | 0,33 | 0,17 |
| n-Butan | 36,24 | 0,71 | 84,36 | 21,89 | 89,30 | 33,45 |
| trans-Buten-2 | 8,84 | 0,11 | 0,55 | 27,27 | 0,54 | 0,23 |
| 1-Buten | 7,18 | 0,03 | 4,20 | 23,59 | 4,41 | 1,93 |
| iso-Buten | 0,10 | 0,00 | 0,04 | 0,27 | 0,04 | 0,02 |
| cis-Buten-2 | 6,73 | 0,18 | 0,69 | 23,15 | 0,65 | 0,26 |
| Butadien | 1,09 | 0,02 | 0,06 | 3,82 | 0,06 | 0,03 |
| | | | | | | |
| Menge, mol/h | 42,46 | 16,27 | 13,98 | 12,21 | 12,89 | 1,09 |

### Beispiel 2

Einer Absorptionskolonne 3 wird im unteren Bereich ein Wasserstoff, Stickstoff, Sauerstoff, n-Butan, Butene und Butadien enthaltender Stoffstrom 1 zugegeben. Im oberen Bereich der Absorptionskolonne wird als Lösungsmittel N-Methylpyrrolidon mit 8,3 Gew.-% Wasser aufgegeben. Am Kopf der Kolonne wird ein Sickstoff, Sauerstoff, Wasserstoff und Butan enthaltender Kopfstrom 7 entnommen. Am Sumpf fällt ein N-Methylpyrrolidon, Wasser, Butane, Butene, Butadien und Kohlenstoffdioxid enthaltender Sumpfstrom 9 an. Als Absorptionskolonne wird eine Kolonne mit 20 theoretischen Trennstufen eingesetzt. Die Eingangstemperatur des Lösungsmittels, das mit einem Molenstrom von 9042 kmol/h zugegeben wird, beträgt 40°C. Die Absorption wird bei einer Kopftemperatur von 41 °C, einer Sumpftemperatur von 63°C und einem Druck von 10 bara durchgeführt. Das Molenstromverhältnis von Lösungsmittel zu zugegebenem Wasserstoff, Stickstoff, n-Butan, Butene und Butadien enthaltendem Stoffstrom 1 liegt bei 2,7.

Der der Absorptionskolonne entnommene Sumpfstrom 9 wird einer Extraktivdestillationskolonne 11 als Zulauf im mittleren Bereich zugegeben. Im oberen Bereich werden der Extraktivdestillationskolonne 901 kmol/h eines N-Methylpyrrolidon und 8,3 Gew.-% Wasser enthaltenden Lösungsmittels zugegeben. Am Kopf der Extraktivdestillationskolonne fällt ein Kopfproduktstrom 15 an, der Butan und Kohlendioxid sowie in Spuren andere Leichtsieder enthält. Der Rücklaufstrom am Kolonnenkopf beträgt 99,5 kmol/h. Am Sumpf der Extraktivdestillationskolonne wird ein N-Methylpyrrolidon, Wasser, Butane, Butene und Butadien enthaltender Sumpfstrom 17 entnommen. Die Extraktivdestillationskolonne enthält 48 theoretische Trennstufen. Der Strom 9 wird bei der neunundzwanzigsten theoretischen Trennstufe und der Strom 13 bei der sechsundvierzigsten theoretischen Trennstufe zugeführt. Die Eingangstemperatur des Stroms 13 beträgt 35°C. Die Extraktivdestillationskolonne wird mit einer Sumpftemperatur von 103°C, einer Kopftemperatur von 44°C und einem Druck von 4,8 bara betrieben. Das Massenverhältnis von der Absorptionskolonne zugegebenem Lösungsmittel 5 zu dem der Extraktivdestillationskolonne zugegebenen Lösungsmittel 13 liegt bei 10.

Der Sumpfstrom aus der Extraktivdestillationskolonne wird als Zulauf einer Destillationskolonne 19 zugegeben. In der Destillationskolonne wird ein n-Butan, Butene und Butadien enthaltender Kopfstrom 21 und ein N-Methylpyrrolidon und Wasser enthaltender Sumpfstrom 23 gewonnen. Die Destillationskolonne enthält 13 theoretische Trennstufen. Der der Extraktivdestillationskolonne entnommene Sumpfstrom 17 wird bei der elften theoretischen Trennstufe zugegeben. Der Strom 21 wird als gasförmiges olefinreiches Kopfprodukt entnommen. Der Rücklaufstrom am Kolonnenkopf beträgt 919 kmol/h. Der Sumpfstrom 23 wird in einem Wärmetauscher 27 gekühlt und zu den Kolonnen 3 und 11 zurückgeführt (Ströme 5 und 13). Die Destillationskolonne 19 wird mit einer Sumpftemperatur von 171°C, einer Kopftemperatur von 43°C und einem Druck von 4,7 bara betrieben.

Der Kopfproduktstrom 15 der Extraktivdestillationskolonne wird einem Kondensator 20 zugeführt und im Kondensator werden ein im Wesentlichen Kohlendioxid, Wasserstoff und n-Butan enthaltender Strom 18 sowie ein im wesentlichen n-Butan enthaltender Strom 16 entnommen. Der im Wesentlichen n-Butan enthaltende Strom wird bevorzugt in eine Dehydrierung zur Herstellung von Butenen und Butadien zurückgeführt.

Die Zusammensetzung und die jeweils zugeführten beziehungsweise erhaltenen Mengen der einzelnen Stoffströme sind in Tabelle 2 aufgeführt.

**Tabelle 2: Zusammensetzung der Stoffströme einer Aufarbeitung eines Butadien enthaltenden Stoffstroms in mol-%**

| Stoffstrom | 1 | 7 | 15 | 21 | 16 | 18 |
|---|---|---|---|---|---|---|
| Butan | 4,56 | 3,74 | 85,63 | 1,20 | 87,44 | 23,66 |
| 1-Buten | 0,02 | 0,01 | 0,63 | 0,00 | 0,64 | 0,22 |
| C-2-Buten | 0,21 | 0,06 | 0,07 | 1,76 | 0,07 | 0,02 |
| T-2-Buten | 0,33 | 0,15 | 0,75 | 2,07 | 0,77 | 0,21 |
| 1.3-Butadien | 9,25 | 0,01 | 0,04 | 93,16 | 0,04 | 0,01 |
| H₂O | 0,54 | 0,22 | 1,86 | 1,81 | 1,91 | 0,17 |
| N-Methylpyrrolidon | | 0,01 | 0,00 | 0,00 | 0,00 | |
| CO₂ | 1,27 | 1,19 | 11,03 | 0,00 | 9,13 | 75,71 |
| O₂ | 0,02 | 0,02 | | | | |
| H₂ | 0,04 | 0,05 | | | | |
| N₂ | 83,77 | 94,54 | | | | |
| Menge, kmol/h | 3380 | 3002 | 146 | 330 | 43 | 4 |

### Bezugszeichenliste

- 1: Buten und/oder Butadien, Butan, Wasserstoff und ggf. Kohlendioxid enthaltender Stoffstrom
- 3: Waschkolonne
- 5: N-Methylpyrrolidon und Wasser enthaltender Stoffstrom
- 7: Wasserstoff enthaltender Kopfstrom
- 9: Buten und/oder Butadien, Waschflüssigkeit und ggf. Kohlendioxid enthaltender Sumpfstrom
- 11: Extraktivdestillationskolonne
- 13: N-Methylpyrrolidon und Wasser enthaltender Stoffstrom
- 15: Butan enthaltender Kopfstrom
- 16: Butan-reicher Strom
- 17: Buten und/oder Butadien, N-Methylpyrrolidon, Wasser und ggf. Kohlendioxid enthaltender Sumpfstrom
- 18: Kohlendioxid-reicher Strom
- 19: Destillationskolonne
- 20: Kondensator
- 21: Buten und/oder Butadien und Kohlendioxid enthaltender Kopfstrom
- 23: N-Methylpyrrolidon und Wasser enthaltender Sumpfstrom
- 25: Kondensator
- 27: Wärmetauscher

## Patentansprüche

1. Verfahren zur Aufbereitung eines Buten und/oder Butadien, Butan, Wasserstoff und/oder Stickstoff und gegebenenfalls Kohlendioxid enthaltenden Stoffstroms (1), folgende Schritte umfassend:
(a) Absorption des Buten und/oder Butadien, Butan, Wasserstoff und/oder Stickstoff und gegebenenfalls Kohlendioxid enthaltenden Stoffstroms (1) mit einem 80 bis 97 Gew.-% N-Methylpyrrolidon und 3 bis 20 Gew.-% Wasser enthaltenen Gemisch (5) wobei ein N-Methylpyrrolidon, Wasser, Buten und/oder Butadien, Butan und gegebenenfalls Kohlendioxid enthaltender Stoffstrom (9) und ein Wasserstoff und/oder Stickstoff und Butan enthaltender Stoffstrom (7) erhalten werden,
(b) Extraktivdestillation des N-Methylpyrrolidon, Wasser, Buten und/oder Butadien, Butan und gegebenenfalls Kohlendioxid enthaltenden Stoffstroms (9) mit einem 80 bis 97 Gew.-% N-Methylpyrrolidon und 3 bis 20 Gew.-% Wasser enthaltenden Stoffstrom (13), wobei der N-Methylpyrrolidon, Wasser, Buten und/oder Butadien, Butan und gegebenenfalls Kohlendioxid enthaltende Stoffstrom (9) in einen N-Methylpyrrolidon, Wasser, Buten und/oder Butadien enthaltenden Stoffstrom (17) sowie einen im Wesentlichen Butan und gegebenenfalls Kohlendioxid enthaltenden Stoffstrom (15) getrennt wird.
(c) Destillation des N-Methylpyrrolidon, Wasser, Buten und/oder Butadien enthaltenden Stoffstroms (17) in einen im Wesentlichen N-Methylpyrrolidon und Wasser enthaltenden Stoffstrom (23) und einen Buten und/oder Butadien enthaltenden Stoffstrom (21).

2. Verfahren gemäß Anspruch 1, **dadurch gekennzeichnet, dass** der in Schritt (b) erhaltene im Wesentlichen Butan und Kohlendioxid enthaltende Stoffstrom (15) kondensiert wird, wobei das Kohlendioxid abgetrennt wird.

3. Verfahren gemäß Anspruch 1 oder 2, **dadurch gekennzeichnet, dass** zumindest ein Teil des in Schritt (c) abgetrennten N-Methylpyrrolidon und Wasser enthaltenden Stoffstroms (23) in die Absorption und/oder in die Extraktivdestillation zurückgeführt wird.

4. Verfahren gemäß einem der Ansprüche 1 bis 3, **dadurch gekennzeichnet, dass** die Absorption in Schritt (a) bei einer Sumpftemperatur im Bereich von 30 bis 160°C, einer Kopftemperatur im Bereich von 5 bis 60°C und einem Druck im Bereich von 2 bis 20 bar durchgeführt wird.

5. Verfahren gemäß einem der Ansprüche 1 bis 4, **dadurch gekennzeichnet, dass** die Absorption in Schritt (a) in einer Kolonne (3) durchgeführt wird, der der 80 bis 97 Gew.-% N-Methylpyrrolidon und 3 bis 20 Gew.-% Wasser enthaltende Stoffstrom (5) und der Buten und/oder Butadien, Butan, Wasserstoff und Kohlendioxid enthaltende Stoffstrom (1) im Gegenstrom zugegeben werden.

6. Verfahren gemäß einem der Ansprüche 1 bis 5, **dadurch gekennzeichnet, dass** die Extraktivdestillation in Schritt (b) in einer Extraktivdestillationskolonne (11) durchgeführt wird, der der N-Methylpyrrolidon, Wasser, Buten und/oder Butadien, Butan und Kohlendioxid enthaltende Stoffstrom (9) und der 80 bis 97 Gew.-% N-Methylpyrrolidon und 3 bis 20 Gew.-% Wasser enthaltende Stoffstrom (13) im Gegenstrom zugegeben werden.

7. Verfahren gemäß einem der Ansprüche 1 bis 6, **dadurch gekennzeichnet, dass** der in der Absorption in Schritt (a) abgetrennte Wasserstoff enthaltende Stoffstrom (7) in eine Butandehydrierung, der der Buten und/oder Butadien, Butan, Wasserstoff und/oder Stickstoff und Kohlendioxid enthaltende Stoffstrom (1) entstammt, zumindest teilweise zurückgeführt wird.

8. Verfahren gemäß einem der Ansprüche 1 bis 7, **dadurch gekennzeichnet, dass** das in der Extraktivdestillation in Schritt (b) abgetrennte Butan in eine Butandehydrierung zurückgeführt wird.

9. Verfahren gemäß einem der Ansprüche 1 bis 8, **dadurch gekennzeichnet, dass** das Verhältnis von bei der Absorption in Schritt (a) zugegebener Mischung aus Wasser und N-Methylpyrrolidon zu der bei der Extraktivdestillation in Schritt (b) zugegebenen Mischung aus Wasser und N-Methylpyrrolidon im Bereich von 0,2 bis 20 liegt.

## Claims

1. A process for workup of a stream (1) comprising butene and/or butadiene, butane, hydrogen and/or nitrogen, with or without carbon dioxide, comprising the following steps:
(a) absorption of the stream (1) comprising butene and/or butadiene, butane, hydrogen and/or nitrogen, with or without carbon dioxide, with a mixture (5) comprising 80 to 97% by weight of N-methylpyrrolidone and 3 to 20% by weight of water to obtain a stream (9) comprising N-methylpyrrolidone, water, butene and/or butadiene, and butane, with or without carbon dioxide, and a stream (7) comprising hydrogen and/or nitrogen and butane,
(b) extractive distillation of the stream (9) comprising N-methylpyrrolidone, water, butene and/or butadiene, and butane, with or without carbon dioxide, with a stream (13) comprising 80 to 97% by weight of N-methylpyrrolidone and 3 to 20% by weight of water to separate the stream (9) comprising N-methylpyrrolidone, water, butene and/or butadiene, and butane, with or without carbon dioxide, into a stream (17) comprising N-methylpyrrolidone, water, butene and/or butadiene, and a stream (15) comprising essentially butane, with or without carbon dioxide,
(c) distillation of the stream (17) comprising N-methylpyrrolidone, water, butene and/or butadiene into a stream (23) comprising essentially N-methylpyrrolidone and water, and a stream (21) comprising butene and/or butadiene.

2. The process according to claim 1, wherein the stream (15) which comprises essentially butane and carbon dioxide and is obtained in step (b) is condensed to remove the carbon dioxide.

3. The process according to claim 1 or 2, wherein at least a portion of the stream (23) which comprises N-methylpyrrolidone and water and is removed in step (c) is recycled into the absorption and/or into the extractive distillation.

4. The process according to any of claims 1 to 3, wherein the absorption in step (a) is performed at a bottom temperature in the range from 30 to 160°C, a top temperature in the range from 5 to 60°C and a pressure in the range from 2 to 20 bar.

5. The process according to any of claims 1 to 4, wherein the absorption in step (a) is performed in a column (3) to which the stream (5) comprising 80 to 97% by weight of N-methylpyrrolidone and 3 to 20% by weight of water, and the stream (1) comprising butene and/or butadiene, butane, hydrogen and carbon dioxide are added in countercurrent.

6. The process according to any of claims 1 to 5, wherein the extractive distillation in step (b) is performed in an extractive distillation column (11) to which the stream (9) comprising N-methylpyrrolidone, water, butene and/or butadiene, butane and carbon dioxide, and the stream (13) comprising 80 to 97% by weight of N-methylpyrrolidone and 3 to 20% by weight of water are added in countercurrent.

7. The process according to any of claims 1 to 6, wherein the hydrogen-comprising stream (7) removed in the absorption in step (a) is at least partly recycled into a butane dehydrogenation from which the stream (1) comprising butene and/or butadiene, butane, hydrogen and/or nitrogen and carbon dioxide originates.

8. The process according to any of claims 1 to 7, wherein the butane removed in the extractive distillation in step (b) is recycled into a butane dehydrogenation.

9. The process according to any of claims 1 to 8, wherein the ratio of mixture of water and N-methylpyrrolidone added in the absorption in step (a) to the mixture of water and N-methylpyrrolidone added in the extractive distillation in step (b) is in the range from 0.2 to 20.

## Revendications

1. Procédé pour le traitement d'un flux de substances (1) contenant du butène et/ou du butadiène, du butane, de l'hydrogène et/ou de l'azote et le cas échéant du dioxyde de carbone, comprenant les étapes suivantes
(a) absorption du flux de substances (1) contenant du butène et/ou du butadiène, du butane, de l'hydrogène et/ou de l'azote et le cas échéant du dioxyde de carbone par un mélange (5) contenant 80 à 97% en poids de N-méthylpyrrolidone et 3 à 20% d'eau, avec obtention d'un flux de substances (9) contenant de la N-méthylpyrrolidone, de l'eau, du butène et/ou du butadiène, du butane et le cas échéant du dioxyde de carbone et d'un flux de substances (7) contenant de l'hydrogène et/ou de l'azote et du butane,
(b) distillation extractive du flux de substances (9) contenant de la N-méthylpyrrolidone, de l'eau, du butène et/ou du butadiène, du butane et le cas échéant du dioxyde de carbone avec un flux de substances (13) contenant 80 à 97% en poids de N-méthylpyrrolidone et 3 à 20% d'eau, le flux de substances (9) contenant de la N-méthylpyrrolidone, de l'eau, du butène et/ou du butadiène, du butane et le cas échéant du dioxyde de carbone étant séparé en un flux de substances (17) contenant de la N-méthylpyrrolidone, de l'eau, du butène et/ou du butadiène ainsi qu'en un flux de substances (15) contenant essentiellement du butane et le cas échéant du dioxyde de carbone,
(c) distillation du flux de substances (17) contenant de la N-méthylpyrrolidone, de l'eau, du butène et/ou du butadiène en un flux de substances (23) contenant essentiellement de la N-méthylpyrrolidone et de l'eau et en un flux de substances (21) contenant du butène et/ou du butadiène.

2. Procédé selon la revendication 1, **caractérisé en ce que** le flux de substances (15) contenant essentiellement du butane et du dioxyde de carbone obtenu dans l'étape (b) est condensé, le dioxyde de carbone étant séparé.

3. Procédé selon la revendication 1 ou 2, **caractérisé en ce qu'**au moins une partie du flux de substances (23) contenant de la N-méthylpyrrolidone et de l'eau séparé dans l'étape (c) est recyclée dans l'absorption et/ou dans la distillation extractive.

4. Procédé selon l'une quelconque des revendications 1 à 3, **caractérisé en ce que** l'absorption dans l'étape (a) est réalisée à une température du fond dans la plage de 30 à 160°C, à une température de tête dans la plage de 5 à 60°C et à une pression dans la plage de 2 à 20 bars.

5. Procédé selon l'une quelconque des revendications 1 à 4, **caractérisé en ce que** l'absorption dans l'étape (a) est réalisée dans une colonne (3) dans laquelle on introduit, à contre-courant, le flux de substances (5) contenant 80 à 97% en poids de N-méthylpyrrolidone et 3 à 20% en poids d'eau et le flux de substances (1) contenant du butène et/ou du butadiène, du butane, de l'hydrogène et du dioxyde de carbone.

6. Procédé selon l'une quelconque des revendications 1 à 5, **caractérisé en ce que** la distillation extractive dans l'étape (b) est réalisée dans une colonne de distillation extractive (11) dans laquelle on introduit, à contre-courant, le flux de substances (9) contenant de la N-méthylpyrrolidone, de l'eau, du butène et/ou du butadiène, du butane et du dioxyde de carbone et le flux de substances (13) contenant 80 à 97% en poids de N-méthylpyrrolidone et 3 à 20% en poids d'eau.

7. Procédé selon l'une quelconque des revendications 1 à 6, **caractérisé en ce que** le flux de substances (7) contenant de l'hydrogène séparé dans l'absorption dans l'étape (a) est au moins partiellement recyclé dans une déshydrogénation de butane dont provient le flux de substances (1) contenant du butène et/ou du butadiène, du butane, de l'hydrogène et/ou de l'azote et du dioxyde de carbone.

8. Procédé selon l'une quelconque des revendications 1 à 7, **caractérisé en ce que** le butane séparé dans la distillation extractive dans l'étape (b) est recyclé dans une déshydrogénation de butane.

9. Procédé selon l'une quelconque des revendications 1 à 8, **caractérisé en ce que** le rapport du mélange d'eau et de N-méthylpyrrolidone ajouté lors de l'absorption dans l'étape (a) et du mélange d'eau et de N-méthylpyrrolidone ajouté lors de la distillation extractive dans l'étape (b) se situe dans la plage de 0,2 à 20.
